Europäisches Patentamt

European Patent Office

Office européen des brevets

(11) Publication number: **0 119 085**
B1

# EUROPEAN PATENT SPECIFICATION

(45) Date of publication of patent specification: 07.12.88

(51) Int. Cl.⁴: **A 61 B 5/00**, G 01 N 21/85

(21) Application number: 84301629.6

(22) Date of filing: 09.03.84

(54) Photoelectric brain scanner and its use.

(30) Priority: 10.03.83 JP 35108/83 u
10.03.83 JP 35109/83 u
10.03.83 JP 35110/83 u

(43) Date of publication of application:
19.09.84 Bulletin 84/38

(45) Publication of the grant of the patent:
07.12.88 Bulletin 88/49

(84) Designated Contracting States:
CH DE FR IT LI NL SE

(56) References cited:
EP-A-0 047 094
EP-A-0 063 431
EP-A-0 093 927
GB-A-2 108 675
US-A-3 811 777
US-A-3 817 249

(73) Proprietor: SHIONOGI & CO., LTD.
12, Dosho-machi 3-chome Higashi-ku
Osaka 541 (JP)

(72) Inventor: Ikeda, Masato
21-14, 3-chome, Shikanodainishi
Ikoma-shi Nara (JP)
Inventor: Kurokawa, Kenji
4-1, 2-chome, Shinwadai Tarumi-ku
Kobe-shi Hyogo (JP)
Inventor: Yoshida, Norihiko
27-16, 2-chome, Himuro-cho
Takatsuki-shi Osaka (JP)
Inventor: Miyazaki, Hiroshi
80-24, Nimyo-cho
Nara-shi Nara (JP)
Inventor: Matsushita, Akira
3-22, 2-chome, Fukaeminami-cho Higashinada-ku
Kobe-shi Hyogo (JP)

(74) Representative: Bizley, Richard Edward et al
BOULT, WADE & TENNANT 27 Furnival Street
London EC4A 1PQ (GB)

Courier Press, Leamington Spa, England.

## Description

The present invention generally relates to a system for scanning the brain tissues of an experimental animal. In particular, it is concerned with an apparatus having, as its essential components, a flexible cord assembly of a coated pair of optical conductors providing a probe for penetrating into brain tissues, a means for driving the probe and a photoelectric processor which provides light to be transmitted into the brain tissues and processes the light signal returned from the tissues to present the scanning results, e.g. as a chart.

It has been suggested to scan the brain tissues of an experimental animal by detecting the difference in reflectance in a particular site of the brain tissue while a pair of glass optical fibres is advanced through the brain. Light is transmitted through one of a pair of the optical fibres to a spot in the tissue where the light is reflected or dispersed. Only the reflected light is picked up at the distal end of the pair and sent back through the other of the pair to be sensed and read. As the tip of the pair of fibres advances, the read-out signal varies accordingly, and may be recorded by any means such as e.g. a pen-writing recorder.

Since the brain tissue usually had characteristically different conditions for light dispersion or reflection at its specific locality, the above-mentioned read-out value or recorded chart, i.e., "brain map", supplied detailed information about the brain tissue. Thus, a spot in the tissue can be specified and made an indicator for conducting subsequent experiments. The spot is a limited brain region where (an) electrode(s) or (a) cannula(e)) is/are accurately placed or embedded.

Drugs which can participate in or induce a biochemical reaction therein may be infused into the spot through the cannula. In addition to the pick-up of spontaneous biological electrical signals from the brain tissue, the embedded electrode may be used to supply the brain with electrical energy which stimulates the nerve fibres or initiates an electrochemical reaction therein.

The fundamental concept of this valuable technique has already been disclosed by the present inventors (see, for instance, Journal of Neuroscience Method, 2, (1980), 9—17). The present inventors have now developed a practically useful system for scanning brain tissues.

It is known from EP—A—0047094 to provide a photoelectric scanner which comprises a flexible cord assembly comprising two optical conductors, providing inward and outward pathways respectively, being coated with optically shielding material, forming a probe at one end of the assembly for penetrating into tissues and having an inlet and outlet plug at the other end of the assembly, the probe comprising a stepped armoring pipe which covers the outside of the optical conductors; and a photoelectric processor including a light source coupled to an inlet to the inward pathway, a photoelectric transducer coupled with an outlet of the outward pathway and producing an electric signal in response to light incident on the transducer, two sockets, each one having a casing to shield out exterior light and for holding one of the plugs, an amplifier adapted to receive as an input the electric signal, a recorder adapted to receive output signals from the amplifier, means for adjusting the gain of the amplifier, and a power source.

According to the invention such a scanner is characterised by means for driving the probe lengthwise and by means for detachably retaining a cannula-guide on the armoring pipe.

The cannula guide is preferably held in position by a pair of clamps. The roots of the clamps may be fixed to the pipe at the root section and the tips thereof may extend to the penetrating section. Since the clamp is usually made of a resilient material it does not hinder the accommodating of the cannula guide but retains the cannula guide once it has been accommodated by pressing inwardly on it. The guide can then rest at the predetermined position of the penetrating section until it is forcibly pulled out. Alternatively, the upper part of the penetrating section of the probe may be expanded to form a lobe or the like and said expanded part is made sufficiently large to retain the inserted guide by outward pressure in order to fulfil the same object.

In a preferred embodiment of the invention, at the other end of the optical conductors of the flexible cord assembly are twin inlet/outlet plugs each covered with an armoring-pipe. This armoring-pipe may also comprise at least two pipes of differing diameters arranged concentrically but offset longitudinally to form a "notched" configuration of which the diameter at the root section is larger than that of the smaller diameter section. Each of the plug armoring-pipes contains a single optical conductor (e.g. a glass fibre). The pair of optical conductors in the flexible cord assembly branch apart near said plug positions. The branched position and joints between the armoring-pipes and the flexible cord running through the armoring-pipes are usually reinforced by sheaths having approximately the same flexibility as that of the optical conductor. The material for this purpose may be, e.g., a thermal-shrinking polyvinyl chloride.

In the photoelectric processor, both the light source and the photoelectric transducer are usually positioned such that they are functionally directed substantially upwardly (preferably in an upright or vertical direction) and are covered with sockets having cylindrical casings which stand upright thereon. Each of the sockets has a centre guide tube for holding the respective plug vertical while permitting a margin of angular displacement for the plug. The socket is high enough to guide the plug tip down to the focussing region of the light source or to the light receiving surface of the photoelectric transducer. This object can effectively be attained by approximating the depth of the throughhole through the centre guide tube down to said region or to the surface to the length of the small diameter section of the plug armoring-pipe.

The light source may be an incandescent lamp (preferably with an interior lens) a light emitting diode or a semiconductor laser device, and a photomultiplier or a semiconductor photo detector may be used as the photoelectric transducer.

In practice, only a fraction (e.g. barely one tenth) of the whole light flux produced can be transmitted along the optical conductor and a fraction (e.g. barely only one tenth) of the light receiving surface can be utilized for receiving the light incident thereon. This drawback can however be offset by amplifying the fractional photoelectric current by means of an internal amplifier for the photoelectric transducer and by a subsequent amplifier.

What is important here is the centering of the optical axis of the light source to the axis of the optical conductor and maintaining the light receiving surface perpendicular to the axis of the optical conductor while shielding these optical connections from exterior light. It is also important to design these components so that the connection of the plug with and disconnection of that from the photoelectric processor can be performed in a simple and easy operation with sufficient accuracy and so that rotating movement of the plug armoring-pipe in the connected state relative to the processor may be performed to a limited extent without adversely affecting the transmission of a light signal therethrough.

The light source and the photoelectric transducer may be driven by a stabilized power source placed in the processor, an electric signal produced in the photoelectric transducer being supplied via an amplifier (containing one or more normal operational amplifiers) to a recorder which may produce a desired chart.

The previously-described extremely thin single optical conductors are usually made of glass and are easy to insert into and pierce through the rigid thin pipes, and no problem results therefrom in the structuring the probe and plugs encased in the rigid armoring-pipe. Thus, the thinnest of commercially available rigid pipes, for instance, a stainless steel thin pipe of about 300—320 µm outside diameter and about 120—150 µm inside diameter, may be selected for structuring these armoring pipes.

Inside the rigid pipe assembly which constitutes the armoring-pipe of the probe, usually two optical conductors are arranged in parallel with each other and glued together with an adhesive agent of the instant hardening type in such a manner that the distal ends are even. Each of the thin pipe assemblies which constitute a plug armoring-pipe encloses a single optical conductor for gluing. By virtue of the previously described stepped configuration, the plugs ensure a stable connection with the light source or with the photoelectric transducer. Thus, by simply inserting the plug armoring-pipes into the centre through holes of the sockets until the shoulders of the steps rest on the upper edges of the respective guide tube, the respective plug reaches the light receiving surface of the photoelectric transducer or the focussing region of the light source, as appropriate.

This accommodated state can be maintained without any additional means for retaining the plugs, and the latter are permitted a limited extent of angular displacement without producing any adverse effect on the optical connections. This feature is of particular importance because the scanning operation inevitably entails occasionally minute movements of an associated implement such as a stereotaxic instrument which carries and holds the animal (e.g. a rat) brain thereon and of a probe driving implement as an attachment to the stereotaxic instrument with respect to the photoelectric processor. Such movement may cause troubles in the plug's accommodated state.

Since the optical conductor enclosed in the flexible cord assembly is extremely thin and fragile if it is made of glass, particular care should be exercised in handling the same. Moreover, the signal/noise ratio may adversely be affected by exterior light if it is used in the naked state. Thus, it is preferred to coat the optical conductor in the flexible segment with a suitable material to protect against mechanical impact. This coating can also give suitable light-insulation in the flexible cord segment. The coating is preferably a flexible tube made of a light insulator of relatively low coefficient of friction in order to facilitate the coating (sheathing) operation. Suitable materials are, e.g., polyolefins such as polyethylene and fluorine-containing polymers such as polytetrafluoroethylene.

The branched position of the flexible segment of the cord and the joints between the armoringpipes and the flexible coating are preferably reinforced by sheaths having approximately the same flexibility as that of the optical conductor in order to give sufficient deflective strength to the conductor. A suitable material for this is preferably polyvinyl chloride containing a controlled amount of plasticizer, particularly, a thermalshrinking vinyl resin such as that conventionally used for cylindrical solid resistors or wound-type film capacitors.

In accordance with the present invention, the armoring-pipe of the probe is structured to retain a detachable cannula-guide or other guide at least temporarily. The term "cannula-guide" as used throughout this specification including the claims should be broadly construed such that the function of the guide is not necessarily restricted to the guiding of a cannula or cannulae, but may have other or alternative guiding functions useful to those skilled in the art. The guide may also be a simple thin stainless steel pipe with both ends open, similar to that used for the armoring pipe.

During the scanning operation, the detachable cannula guide accomodated on the penetrating section of the armoring-pipe may be glued to the cranial bones around its periphery with dental cement or the like, and when the operation is completed by pulling the probe out from the brain the guide is then left unmoved on the cranial bones as a cannula guide which may subsequently be used in accurately positioning a cannula for e.g. drug infusion.

The present invention will now be described and illustrated in more detail by referring to preferred embodiments shown in the attached drawings, in which most of the components are designated by the same reference numerals throughout the drawings.

Fig. 1, not intended to represent an embodiment of the invention but included to show how the invention is put in practice, is a perspective view schematically showing the entire arrangement of a photoelectric brain scanner together with associated implements.

Fig. 2, not intended to represent an embodiment of the invention but included to show how the invention is put in practice, is a side sectional view of sockets and associated components inside the housing of the photoelectric processor seen in Fig. 1.

Fig. 3, not intended to represent an embodiment of the invention but included to show how the invention is put in practice, is a schematic view showing the flexible cord assembly of the scanner shown in Fig. 1.

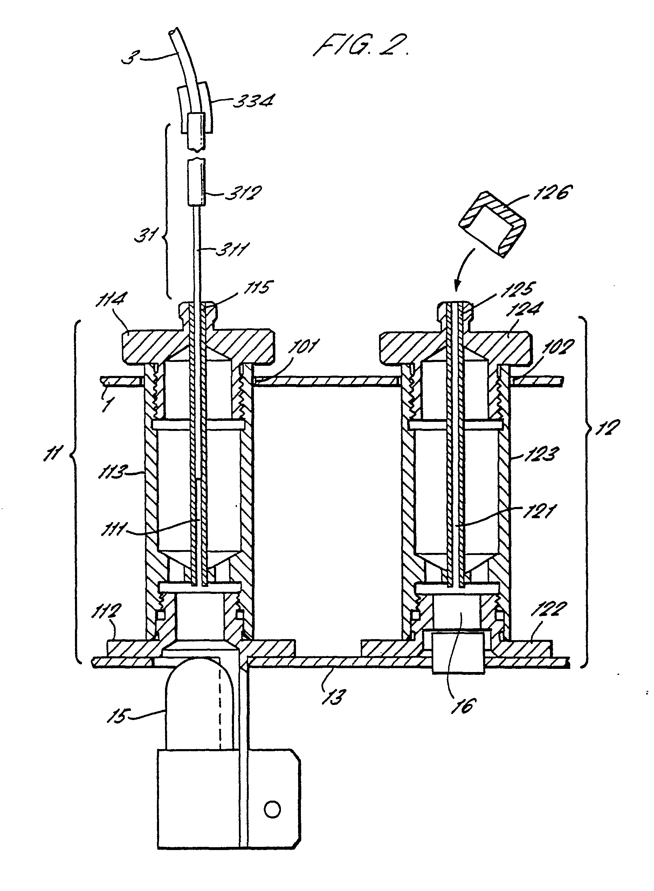

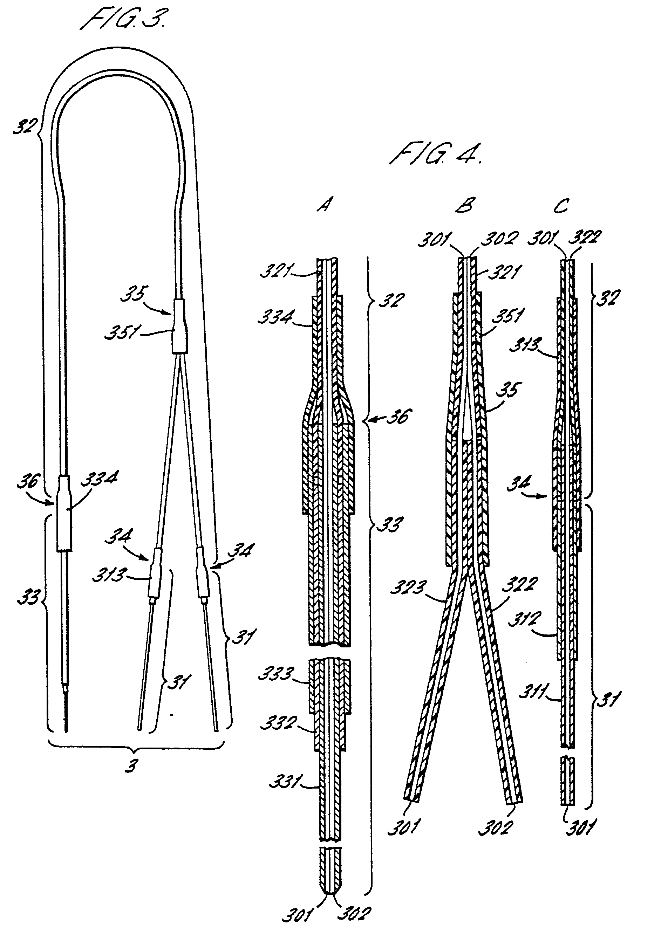

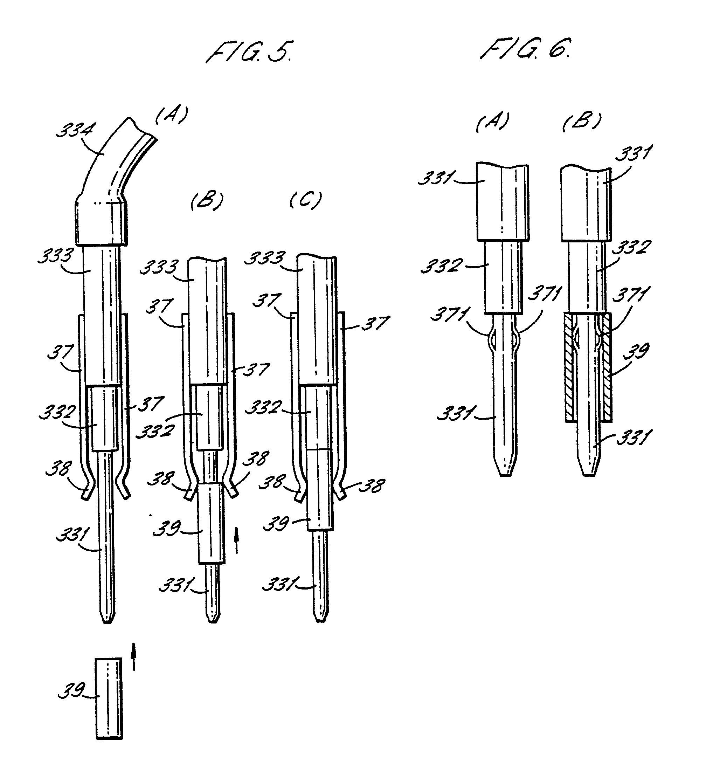

Each of Figs. 4A, 4B and 4C, not intended to represent an embodiment of the invention but included to show how the invention is put in practice, is a sectional view showing the parts which constitute the flexible cord assembly, 4A specifically representing the probe for penetrating into brain tissue, 4B representing the branched position of the flexible cord, and 4C representing the plug, respectively.

Each of Figs. 5A, 5B and 5C is an enlarged schematic view of the armoring-pipe of the probe with a clamp means built in accordance with a preferred embodiment and showing the manner of accommodating the cannula guide thereto at varying stages of the accommodating or detaching operation.

Finally, Figs. 6A and 6B are enlarged partial schematic views representing the section of the probe encased in the armoring-pipe equipped with an alternative for retaining the cannula guide.

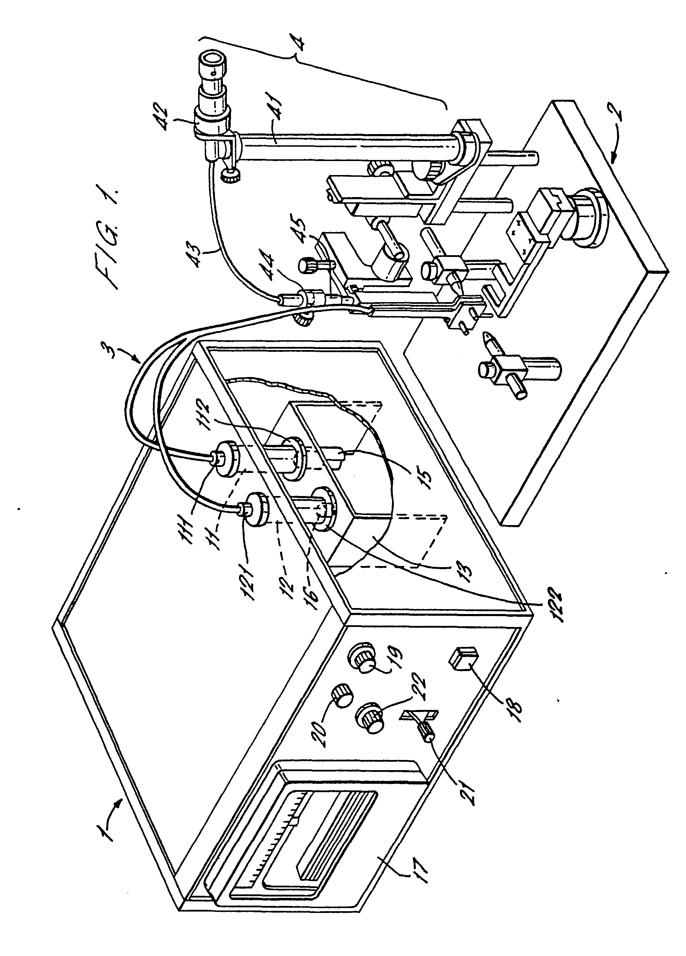

In Fig. 1 which illustrates the entire arrangement for the brain tissue scanning operation, there is a stereotaxic instrument 2 which may be any commercially available type, a photoelectric processor 1, a flexible cord assembly 3 and means 4 for driving the probe which, in combination, constitute a photoelectric brain scanner.

In the photoelectric processor 1, there are twin sockets 11 and 12 having centre-guide-tubes 115 and 125 which provide through holes 111 and 121 for receiving twin plugs 31 of the flexible cord assembly 3, respectively. Both the sockets 11 and 12 are mounted on an abutment 13 placed in the housing of the processor 1 in the proximity of the side wall covering a light source 15 (an incandescent lamp, LNS-SB12-71X, available from Hamai Denkyu K.K.) and a photoelectric transducer 16 (Optical detector, 509—50, available from Bell Howell Co., Ltd.).

On the front panel of the housing of the processor 1, is a pen-writing recorder 17 (SR 6402, available from Watanabe Sokki K.K.) and a mains power switch 18 which also serves as a working indicator. Numeral 19 represents a knob for gain adjustment of the amplifier (not shown) and numeral 20 indicates a knob for zero-point adjustment of the recorder 17. Numeral 21 represents a change-over switch for the means for driving the probe (probe driving means) 4 which functions as the power supply control as well as the driving direction selector and numeral 22 represents a knob for adjusting the driving speed.

The probe driving means 4 consists of a reduction geared motor 42 detachably mounted on a commercially available stereotaxic instrument 2 for the rat brain (Takahashi Shoten) through a bracket 41, a rotating adaptor 44 attached to the axis of the fine adjustment device 45 of the stereotaxic instrument 2 and a flexible shaft 43 which couples said rotating adaptor 44 with the rotating shaft of the motor 42, which is supplied with electric current from the main circuitry in the processor 1 under the control of switch 21 and knob 22.

Fig. 2 shows details of the sockets 11 and 12 together with their associated components. Cylindrical casings 113 and 123 are threaded into flanges 112 and 122 fastened to the abutment 13 at their bottom ends, while their top ends project from the ceiling (top panel) of the housing of the processor 1 through through holes 101 and 102 and are closed with lids 114 and 125, respectively, which are secured to the casings by threading.

Piercing through the centres of these structural elements, guide-tubes 115 and 125 are fixed in the casings 113 and 123. The guide-tubes 115 and 125, in turn, provide through holes 111 and 121 for accommodating the plugs 31 of the flexible cord assembly 3.

The socket 11 shown in the left side of Fig. 2 is shown with plugs 31 inserted midway. Since the plug 31 has a stepped configuration and the length of the smallest diameter section 311 is adjusted as previously described, the tip of the plug 31 reaches the focussing region of the light source 15 when the plug 31 is inserted until the shoulder of the notch touches the top edge of the guide tube 115. In the case of the right side socket 12, wherein the photoelectric transducer 16 is shown, the length is so adjusted that the distal end of the optical conductor may be brought in close contact with the light receiving surface of the photoelectric transducer 16. A cap schematically shown and referenced by numeral 126 may be provided to cover the top open end of the guide tube 125 in the non-used state and a similar means may also be provided in the left side socket to cover the open end of the guide tube 115.

In this embodiment, since the sockets 11 and 12 comprise flanges 112 and 122, the guide-tubes 115 and 125, the casings 113 and 123, and the lids 114 and 124 for easy assembly and disassembly, cleaning operations for the light source 15 and the upper surface of the photoelectric transducer 16 as well as detaching operations for the top wall (ceiling) of the processor 1 are also made very easy. However, these casings, guide-tubes and lids may be structured integrally as a solid body.

What is essential is the functions of accurately guiding the plugs 31 to direct them to the light source 15 or the optical transducer 16 and of maintaining stable optical connections between them in the accomodated state while permitting a limited extent of angular displacement.

The flexible cord assembly 3 generally shown in Fig. 3 comprises of a probe 33 for penetrating into the brain tissues, twin plugs 31 and a flexible cord 32 which connects the probe 33 with the terminals 31. In addition to the flexible and light-insulating coating 321 and 322 which covers the flexible cord 32 of the assembly 3, there are sheaths 313 and 334 near the joints 34 and 36 and sheath 351 near the branched position 35 of the flexible cord 32.

The cross-sectional views of Figs. 4A, 4B and 4C illustrate the details of the parts which constitute the flexible cord assembly. In the probe 33 shown in Fig. 4A, a pair of glass fibres 301 and 302 are enclosed in a multiply-notched armoring-pipe comprising thin stainless steel pipes 331, 332 and 333 concentrically arranged and glued together with glass fibres 301 and 302.

The thinnest pipe 331 usually has an outside diameter of about 320 μm, and the other pipes 332 and 33, have correspondingly larger diameters so that they can be set in close fitting. At the root section, the intermediate diameter pipe 332 may be shorter than the other pipes 331 and 333 so that the tip of the flexible and light-shielding coating 321 can suitably be sandwiched between the butt ends of the pipes 331 and 333.

The region 36 where the flexible coating joins the armoring-pipe is reinforced by protective sheath 334. At the branched position 35 shown in Fig. 4B, the pair of glass fibres 301 and 302 and the flexible and light-shielding coating 321 are likewise separated into twin single glass fibres 301 and 302, respectively covered with coatings 323 and 322.

These components near the branched position 35 are also reinforced by the protective sheath 351.

In the plug 31, only one of which is shown in Fig. 4C for brevity, the glass fibre 301 is enclosed in an armoring-pipe comprising a stainless steel pipe of smaller diameter 311 and one of large diameter 312 concentrically arranged but offset longitudinally with respect to each other to form a notched configuration. The construction thereof is similar to but simpler than that of the probe 33 and all of the components at the joints 34 are reinforced by a protective sheath 313. Also at this joint 34 the tip of smaller diameter stainless steel pipe 312 and the coating 322 are butted together and sandwiched between the smaller diameter pipe 311 and sheath 313.

Figs. 5A, 5B and 5C show an embodiment illustrating a preferred aspect of the present invention wherein a pair of clamps 37 is associated with the probe 33 and are used to retain a detachable cannula guide 39.

In Fig. 5A, a cannula guide 39 in the form of a simple stainless steel pipe with both ends open is shown to be ready for accomodation around the periphery of the smallest diameter section 331 of the probe 33, the direction of the guide's movement being indicated by an arrow. In Fig. 5B, the guide 39 is already accomodated midway around the section but does not reach its rest position since it is slightly blocked by the tips 38 of clamps 37. In Fig. 5C, the guide 39 is placed at a rest position adjacent to the lower notch, i.e., the butt end of the pipe 332, where the guide 39 is pressed inwards by the tips 38 of the pair of resilient clamps 37 attached to the root section of the pipe 333 by soldering or like means.

At the rest position, the guide 39 may be glued to the cranial bones of the experimental animal with e.g. dental cement, and when a searching operation is completed by pulling the probe 3 out from the brain guide 39 is left glued to the bones by reversing the order of events shown in Figs. 5A, B and C.

Figs. 6A and 6B illustrate another embodiment of the guide retaining means, wherein at least one lobe 371 is provided on the penetrating section 331 of the probe armoring-pipe 33. Fig. 6B shows the state wherein the guide 39 is rested at a position adjacent to the lower notch and retained there by being subjected to outward pressure by the lobe 371. The function of the lobe 371 may obviously be provided for by any expanded part provided on the penetrating section 331.

## Claims

1. A phototelectric scanner which comprises a flexible cord assembly (3) comprising two optical conductors (301, 302), providing inward and outward pathways respectively, being coated with optically shielding material, forming a probe (33) at one end of the assembly for penetrating into tissues and having an inlet and outlet plug (31) at the other end of the assembly, the probe comprising a stepped armoring pipe which covers the outside of the optical conductors; and a photoelectric processor including a light source (15) coupled to the inlet plug of the inward pathway, a photoelectric transducer (16) coupled to the outlet plug of the outward pathway and producing an electric signal in response to light incident on the transducer, two sockets, (11, 12) each one having a casing to shield out exterior light and for holding one of the plugs, an amplifier adapted to receive as an input the electric signal, a recorder (17) adapted to receive output signals from the amplifier, means for adjusting the gain of the amplifier, and a power source; characterised by means (4) for driving the probe lengthwise and by means (37 or 371) for detachably retaining a cannula-guide on the armoring pipe.

9      EP 0 119 085 B1      10

2. A scanner according to claim 1, wherein the means for retaining the cannula-guide comprises a pair of resilient clamps (37).

3. A scanner according to claim 1, wherein the means for retaining the cannula-guide comprises at least one lobe (371) formed on the armoring pipe.

4. A scanner according to any of claims 1 to 3, wherein the inlet and outlet plugs (31) each comprise an armoring pipe which covers a tip portion of a respective one of the optical conductors, which branch apart near the plugs (31).

5. A scanner according to claim 4, wherein the flexible cord assembly includes sheaths (314, 334) made of a material having approximately the same flexibility as that of the optical conductors in the region of the branching apart of the said conductors and around joints between the armoring pipes and the said conductors.

6. A scanner according to any of claims 1 to 5, wherein the light source (15) and the photoelectric transducer (16) are functionally positioned in a substantially upward direction in the photoelectric processor.

7. A scanner according to any of claims 1 to 6, wherein each of the sockets (11, 12) has a casing (113, 123) respectively to cover the light source or the photoelectric transducer and a centre guide tube for holding the respective plug in position whilst permitting angular displacement of the respective plug.

8. A scanner according to any of claims 1 to 7 wherein each socket (11, 12) is positioned respectively either to guide a plug tip down to the focussing region of the light source (15) or to the light receiving surface of the photoelectric transducer (16).

9. A scanner according to any of claims 1 to 8 wherein the means (4) for driving the probe comprises, on a stereotaxic instrument (2), a reduction geared motor (42), a rotating adaptor (44) attached to the axis of a fine adjustment device (45) of the stereotaxic instrument, and a flexible shaft (43) which couples the rotating adaptor with the motor.

**Patentansprüche**

1. Photoelektrischer Scanner mit einer flexiblen Kabelanordnung (3) mit zwei optischen Leitern (301, 302), die einen Eingangs- bzw. Ausgangsweg bilden, und die mit optisch abdichtendem Material beschichtet sind, die eine Sonde (33) an einem Ende der Anordnung bilden zum Eindringen in Gewebe, und die am anderen Ende der Anordnung einen Eingangs- und Ausgangsstecker (31) aufweisen, wobei die Sonde ein gestuftes Bewehrungsrohr aufweist, welches die Außenseite der optischen Leiter bedeckt; und mit einem photoelektrischen Prozessor mit einer Lichtquelle (15), welche mit dem Eingangsstecker des Eingangsweges verbunden ist, einem photoelektrischen Umsetzer (16), der mit dem Ausgangsstecker des Ausgangsweges verbunden ist und ein elektrisches Signal erzeugt in Antwort auf den Umsetzer einfallendes Licht, mit zwei Buchsen (11, 12), die jeweils ein Gehäuse aufweisen, um Außenlicht abzuschirmen, zum Halten jeweils eines Steckers, mit einem Verstärker, der als Eingang das elektrische Signal empfängt, mit einem Aufzeichner (17), der die Ausgangssignale des Verstärkers empfängt, mit einer Vorrichtung zum Einstellen der Verstärkung des Verstärkers und einer Spannungsversorgung, gekennzeichnet durch eine Vorrichtung (4) zum Antreiben der Sonde in Längsrichtung und durch eine Vorrichtung (37 oder 371) zum lösbaren Halten einer Kanülenführung auf dem Bewehrungsrohr.

2. Scanner nach Anspruch 1, dadurch gekennzeichnet, daß die Haltevorrichtung für die Kanülenführung ein Paar von elastischen Klammern (37) aufweist.

3. Scanner nach Anspruch 1, dadurch gekennzeichnet, daß die Haltevorrichtung für die Kanülenführung mindestens eine Nocke (371) aufweist, die auf dem Bewehrungsrohr ausgebildet ist.

4. Scanner nach einem der Ansprüche 1 bis 3, dadurch gekennzeichnet, daß die Eingangs- und Ausgangsstecker (31) jeweils ein Bewehrungsrohr aufweisen, welches einen spitzen Teil des jeweiligen optischen Leiters bedeckt, und welche sich nahe der Stecker (31) verzweigen.

5. Scanner nach Anspruch 4, dadurch gekennzeichnet, daß die flexible Kabelanordnung Umhüllungen (314, 334) aufweisen aus einem Material, das ungefähr die gleiche Elastizität hat wie die optischen Leiter in dem Bereich, wo sich die Leiter verzweigen, und um die Verbindungen zwischen den Bewehrungsrohren und den Leitern.

6. Scanner nach einem der Ansprüche 1 bis 5, dadurch gekennzeichnet, daß die Lichtquelle (15) und der photoelektrische Umsetzer (16) funktionell angeordnet sind in einer im wesentlichen Aufwärtsrichtung im photoelektrischen Prozessor.

7. Scanner nach einem der Ansprüche 1 bis 6, dadurch gekennzeichnet, daß jeder der Sockel (11, 12) ein Gehäuse (113, 123) aufweist, um jeweils die Lichtquelle oder den photoelektrischen Umsetzer zu bedecken, und ein zentrales Führungsrohr zum Halten des jeweiligen Steckers in Stellung, während es eine Winkelversetzung des jeweiligen Steckers zuläßt.

8. Scanner nach einem der Ansprüche 1 bis 7, dadurch gekennzeichnet, daß jeder Sockel (11, 12) so angeordnet ist, daß er jeweils eine Steckerspitze zum Fokusierungsbereich der Lichtquelle (15) bzw. zur Lichtaufnahmefläche des photoelektrischen Umsetzers (16) führt.

9. Scanner nach einem der Ansprüche 1 bis 8, dadurch gekennzeichnet, daß die Vorrichtung (4) zum Antreiben der Sonde bei einem stereotaktischen Instrument (2) aufweist einen Reduktionsgetriebemotor (42), einen Rotationsadapter (44), der auf der Achse eines Feineinstellgerätes (45) des stereotaktischen Elementes befestigt ist, und eine flexible Welle (43), die den Rotationsadapter mit dem Motor verbindet.

6

**Revendications**

1. Dispositif photoélectrique d'exploration comportant un assemblage (3) de cordon flexible comprenant deux conducteurs optiques (301, 302), formant des trajets vers l'intérieur et vers l'extérieur respectivement, revêtus d'un matériau constituant un écran optique, formant une sonde (33) à un extrémité de l'assemblage en vue de pénétrer dans des tissus et possédant une fiche d'admission et de sortie (31) à l'autre extrémité de l'assemblage, la sonde comportant un tube de blindage étagé qui recouvre l'extérieur des conducteurs optiques; et un processeur photoélectrique comprenant une source de lumière (15) couplée à la fiche d'entrée du trajet vers l'intérieur, un transducteur photoélectrique (16) relié à la fiche de sortie du trajet vers l'extérieur et délivrant un signal électrique en réponse à l'incidence de lumière sur le transducteur, deux douilles (11, 12) possédant chacune un boîtier protecteur contre la lumière extérieur et pour supporter une des fiches, um amplificateur destiné à recevoir en tant qu'entrée le signal électrique, un enregistreur (17) destiné à recevoir des signaux de sortie de l'amplificateur, des moyens en vue de régler le gain de l'amplificateur, et une source d'alimentation; caractérisé par des moyens (4) en vue d'entraîner la sonde longitudinalement et par des moyens (37 ou 371) en vue de retenir de façon amovible un guide-canule sur le tube de blindage.

2. Dispositif d'exploration selon la revendication 1, dans lequel les moyens en vue de retenir le guide-canule comportent une paire d'attaches élastiques (37).

3. Dispositif d'exploration selon la revendication 1, dans lequel les moyens en vue de retenir le guide-canule comportent au moins un lobe (371) réalisé sur le tube de blindage.

4. Dispositif d'exploration selon l'une quelconque des revendications 1 à 3, dans lequel les fiches d'entrée et de sortie (31) comportent chacune un tube de blindage qui recouvre une partie d'extrémité d'un des conducteurs optiques respectifs, qui se ramifient près des fiches (31).

5. Dispositif d'exploration selon la revendication 4, dans lequel l'assemblage de cordon flexible comprend des gaines (314, 334) reéalisées en un matériau ayant approximativement la même flexibilité que celui constituant les conducteurs optiques dans la région de la ramification desdits conducteurs et autour de joints entre les tubes de blindage et lesdits conducteurs.

6. Dispositif d'exploration selon l'une quelconque des revendications 1 à 5, dans lequel la source de lumière (15) et le transducteur photoélectrique (16) sont fonctionnellement positionnés dans une direction sensiblement verticale dans le processeur photoélectrique.

7. Dispositif d'exploration selon l'une quelconque des revendications 1 à 6, dans lequel chacune des douilles (11, 12) possède un boîtier (113, 123) respectivement pour recouvrir la source de lumière du transducteur photoélectrique et un tube de guidage central en vue de maintenir la fiche respective en position tout en permettant un déplacement angulaire de la fiche respective.

8. Dispositif d'exploration selon l'une quelconque des revendications 1 à 7, dans lequel chaque douille (11, 12) est positionnée respectivement soit pour guider une extrémité de fiche vers le bas vers la région de concentration de la source de lumière (15) soit vers la surface recevant la lumière du transducteur photoélectrique (16).

9. Dispositif d'exploration selon l'une quelconque des revendications 1 à 8, dans lequel les moyens (4) pour entraîner la sonde comportent, sur un instrument stéréotaxique (2), un moteur (42) muni d'un engrenage réducteur, un adaptateur rotatif (44) fixé à l'axe d'un dispositif de réglage fin (45) de l'instrument stéréotaxique, un arbre flexible (43) qui relie l'adaptateur rotatif au moteur.

FIG. 1.

FIG. 2.

# FIG.3.

# FIG.4.

A        B        C

## FIG. 5.

### (A)

334

333

37

332

37

38

331

### (B)

333

37

37

332

38 38

39

331

### (C)

333

37

37

332

38 38

39

331

39

## FIG. 6.

### (A)

331

332

371

331

### (B)

331

332

371 371

39

331